# EUROPEAN PATENT SPECIFICATION

(11) **EP 3 154 559 B1**
(45) Date of publication and mention of the grant of the patent: **01.09.2021**
(21) Application number: 15730858.6
(22) Date of filing: 10.06.2015
(51) Int. Cl.: A61K 38/00, A61K 38/22, A61B 5/00, A61P 9/00, A61P 11/00, A61P 9/04

(54) **USE OF SERELAXIN TO REDUCE GDF-15**
VERWENDUNG VON SERELAXIN ZUR REDUZIERUNG VON GDF-15
UTILISATION DE SÉRÉLAXINE POUR RÉDUIRE LE GDF-15

(30) Priority: 13.06.2014 US 201462011744 P
(43) Date of publication of application: 19.04.2017
(73) Proprietor: Novartis AG, 4056 Basel (CH)
(72) Inventor: PRESCOTT, Margaret Forney, East Hanover, New Jersey 07936-1080 (US); ZHANG, Yiming, East Hanover, New Jersey 07936-1080 (US); DAHLKE, Marion, CH-4056 Basel (CH); SEVERIN, Thomas, 4056 Basel (CH)
(74) Representative: Larbig, Karen Dorothee
(86) International application number: PCT/IB2015/054399
(87) International publication number: WO 2015/189790

(56) References cited:
- WO-A1-2013/004607
- US-A1- 2010 048 475
- KAI C WOLLERT ET AL: "Growth Differentiation Factor 15 in Heart Failure: An Update", CURRENT HEART FAILURE REPORTS, CURRENT SCIENCE INC, NEW YORK, vol. 9, no. 4, 9 September 2012 (2012-09-09), pages 337-345, XP035134728, ISSN: 1546-9549, DOI: 10.1007/S11897-012-0113-9
- FREDRIK E. WIKLUND ET AL: "Macrophage inhibitory cytokine-1 (MIC-1/GDF15): a new marker of all-cause mortality", AGING CELL, vol. 9, no. 6, 21 October 2010 (2010-10-21), pages 1057-1064, XP055204078, ISSN: 1474-9718, DOI: 10.1111/j.1474-9726.2010.00629.x
- None

## Description

### FIELD

The invention relates to the field of therapeutic intervention in cardiovascular disease. More particularly, it relates to growth differentiation factor 15, a pro-inflammatory peptide. It further relates to serelaxin, a hormone recently shown to be effective in treating heart failure.

### BACKGROUND

Heart failure is a disease in which the heart is unable to supply enough blood to meet the body's needs. It is the leading cause of hospitalization in people 65 years of age or older and the prevalence is expected to rise as the population ages and survival rates following myocardial infarction improve. Acute heart failure is associated with a high risk of in-hospital and post-discharge mortality due to the rapid onset or change in the signs and symptoms of heart failure. Patients with pulmonary arterial hypertension present with a sustained elevation of pulmonary arterial pressure and a low mean capillary wedge pressure and left ventricular end diastolic pressure. Pulmonary arterial hypertension can be idiopathic (primary) or can develop in the setting of other disorders. The main vascular changes are vasoconstriction, thrombosis and proliferation of smooth muscle and endothelial cells. An imbalance of vasodilation and vasoconstriction is the result of pulmonary endothelial cell dysfunction or injury. Consequently, the lung develops intimal fibrosis, increased medial thickness, pulmonary arteriolar occlusions and plexiform lesions. Pulmonary arterial hypertension is often an end-stage manifestation of pathological conditions, including heart failure, collagen vascular disease, portal hypertension and HIV. Growth differentiation factor 15 (GDF-15) is a member of the TGF-beta superfamily. TGF-beta family members have pleiotropic effects on cell motility and adhesion, cell cycle and inflammation. GDF-15 is a stress-responsive cytokine known to have a role in regulating inflammatory and apoptotic pathways in injured tissues and during disease, as well as having general antiinflammatory and immunosuppressive properties. It is not normally expressed in most tissues except when induced by pathological processes. GDF-15 is up-regulated following acute injury to the heart, liver, kidney, and lung and its elevations at baseline have been shown to be associated with worse outcomes in chronic heart failure, while its increases over time in heart failure patients were shown to be associated both with worsening of echocardiographic parameters and adverse outcomes (Wang et al., Biomarkers 15:671 (2010)). Elevated GDF-15 was recently suggested in some preliminary studies to be no less predictive of long-term mortality than other biomarkers such as NTproBNP, hsCRP, Galectin 3 or hsTnT (Lok et al., Lancet 381:29-39 (2013)). However, to date no studies have previously evaluated GDF-15 in patients with acute heart failure (AHF).

While GDF-15 has been suggested as a marker for the risk of heart failure, its role in cardiovascular disease remains unknown. Many agents induce GDF-15 expression via multiple pathways. The available data fail to establish a role for GDF-15 in the diagnosis, prognosis or treatment of cardiovascular disease.

Despite efforts to determine whether current therapies can reduce the risk of elevated GDF-15 in cardiovascular disease, the factors regulating GDF levels remain unclear. Findings that current therapies increase, decrease and produce no effect on GDF-1 have all been reported. For example, the PLATO trial found that treating patients with non-ST-elevation acute coronary syndrome lowered their GDF-15 levels, regardless of whether the treatment was invasive or non-invasive and regardless of whether they were given clopidogrel or ticagrelor (Wallentin et al., Circulation 129:293 (2014)). Left ventricular assist devices implanted in end stage heart failure patients in order to provide volume and pressure unloading of the left ventricle also lowered their GDF-15 levels (Lok et al, Eur J Heart Failure 14:1249-1256 (2012)). Also, optimizing beta-blocker therapy decreased GDF-15 in systolic chronic heart failure patients (Apostolovic et al., J Heart Failure 12:Suppl 1, S200 (2013)).

Conversely, optimizing beta-blocker therapy was observed to increase GDF-15 in diastolic chronic heart failure patients and was observed to have no effect on systolic chronic heart failure patients (Eur J Heart Failure 11 :Suppl 1, S45 (2012)). Valsartan treatment of patients with symptomatic heart failure had no effect on GDF-15 but was observed to lower the heart failure biomarker B-type natriuretic peptide (Anand et al., Circulation 122:1387-1395 (2010)). Similarly, even though GDF-15 has been detected in atherosclerotic plaque macrophages and infarcted myocardium, statins did not affect GDF-15 levels (Bonaca et al., Arterioscl Thromb Vasc Biol 31:203-210 (2011)).

Recent clinical trials testing tezosentan, levosimendan, tolviptan and rolofylline failed to demonstrate safety and/or efficacy in treating acute heart failure. Even drugs that have been approved, e.g., milrinone, nesiritide and levosimendan, have raised persistent safety concerns. The agents now used to treat acute heart failure have not been substantially added to or improved upon in several decades. Furthermore, none of the drugs that are approved or are in development are associated with GDF-15 levels.

Serelaxin is a recombinant form of human serelaxin-2 (HR-2), a naturally occurring peptide hormone which increases during pregnancy, mediates maternal physiological cardiovascular and renal adaptations, and has potential protective effects on organ damage. Serelaxin binds to the RXFP1 receptor in the renal and systemic vasculature and in the epithelium of the kidney mediating multiple beneficial effects in acute heart failure including increased arterial compliance, cardiac output, and renal blood flow. In a recently completed Phase 3 trial, serelaxin provided rapid relief of dyspnea and reduced mortality at six months in patients with acute heart failure (Teerlink et al., Lancet 381:29-39 (2013).

The inventors have surprisingly found that the use of serelaxin can be used to lower GDF-15 levels in patients with cardiovascular disease, and is particularly useful in lowering GDF-15 levels in patients with acute heart failure. The inventors have also discovered a method of assessing pulmonary load in a patient with pulmonary congestion. Furthermore, the inventors have discovered that GDF-15 provides a biomarker for assessing pulmonary hemodynamics and informs the diagnosis, prognosis and treatment of heart failure.

### SUMMARY OF THE INVENTION

The invention generally provides a method of assessing pulmonary load in a patient with pulmonary congestion, provides a biomarker for assessing pulmonary hemodynamics and informs the diagnosis, prognosis and treatment of heart failure. It provides a method for lowering GDF-15 levels in patients with cardiovascular disease by treating with serelaxin, the first drug therapy to lower GDF-15 levels.

The present invention provides serelaxin for use in selectively reducing the pulmonary load of a patient with acute heart failure (AHF) comprising:
obtaining a biological sample from a patient;
measuring the amount of GDF-15 in the biological sample; and
thereafter administering serelaxin in response to an elevated level of GDF-15, wherein serelaxin is administered subcutaneously at an infusion rate of 3 µg/kg/day to 150 µg/kg/day to maintain a serum concentration of 1 ng/ml to 100 ng/ml in the patient.

The present invention also provides serelaxin for use in a method of determining the prognosis of mortality of a patient with acute heart failure (AHF) comprising
obtaining a first biological sample from a patient;
detecting the level of GDF-15 in the first biological sample;
administering a therapeutically effective amount of serelaxin, wherein serelaxin is administered subcutaneously at an infusion rate of 3 µg/kg/day to 150 µg/kg/day to maintain a serum concentration of 1 ng/ml to 100 ng/ml in the patient;
obtaining a second biological sample from the patient after administration of serelaxin;
detecting the level of GDF-15 in the second biological sample;
comparing the level of GDF-15 in the first biological sample to the second biological sample; and
predicting the probability of survival, wherein a reduced level of GDF-15 in the second biological sample predicts an increased probability of survival.

### BRIEF DESCRIPTION OF THE DRAWINGS

FIG. 1 shows the effect of serelaxin on the geo-mean of GDF-15 levels compared to placebo.
FIGS. 2A-B represent the correlation between GDF-15 and PAP at baseline and after 20 hours respectively.
FIGS. 3A-B represent the correlation between GDF-15 and PVR at baseline and after 20 hours respectively.
FIGS. 4A-B represent the correlation between GDF-15 and NTproBNP at baseline and after 20 hours respectively.

### DETAILED DESCRIPTION OF THE INVENTION

Initially, any reference to biological samples suitable for assay shall be understood to be any samples known in the art and include, but are not limited, to blood, plasma, serum, buffy coat, leukocytes, lymph, sputum, urine, feces, synovial fluid, synovial cells, cerebrospinal fluid, tears, saliva, hair bulb cells, buccal swabs, and tissue samples. One of skill in the art would realize that some samples would be more readily analyzed following a fractionation or purification procedure.

Similarly, assay methods can be any known in the art and include, but are not limited to, immunoassays of any type, e.g., electrochemiluminescent immunoassay, ELISA or Western blot, HPLC, flow cytometry, Southern blot, electrophoresis and polymerase chain reaction.

The term "assaying" refers to the act of identifying, screening, probing or determining which act may be performed by any conventional means. For example, a sample may be assayed for the presence of or to determine the amount of a particular agent by using an immunoassay, imaging, Northern blot, etc. for the purpose of determining whether that agent is present in a sample or determining the amount of the agent in the sample. The terms "assaying" and "determining" contemplate a transformation of matter, e.g., a transformation of a biological sample from one state to another by means of subjecting that sample to physical testing.

Further, as used herein, the terms "assaying' and "determining" are used to mean testing and/or measuring. The phrase "assaying a biological sample from the patient for ... "and the like are used to mean that a sample may be tested, either directly or indirectly, for either the presence or absence of a given agent (e.g., GDF-15, gene, SNP, protein, etc.) or the amount of a particular agent. It is understood that when an amount of an agent denotes one probability and a different amount of the agent denotes another probability, the amount of the agent may be used to guide a diagnostic, prognostic or therapeutic decision. It is understood that when the presence of an agent denotes one probability and the absence of the agent denotes another probability, either the presence or the absence of the agent may then be used to guide a diagnostic, prognostic or therapeutic decision.

As used herein, "selection," "selectively," "selecting" and "selected" in reference to a patient is used to mean that a particular patient is specifically chosen from a larger group of patients on the basis of the patient having a predetermined criterion. Similarly, "selectively treating" refers to that patient being specifically chosen from a larger group of patients on the basis of the chosen patient having a predetermined criterion. "Selectively administering" refers to administering a drug to a patient specifically chosen from a larger group of patients on the basis of the chosen patient having a predetermined criterion. By "selecting," "selectively treating" and "selectively administering," it is meant that a patient is delivered a personalized therapy based on the patient's particular biology, rather than being delivered a standard treatment regimen based solely on the patient having a particular disease. Selecting does not refer to the fortuitous diagnosis, prognosis or treatment of a patient but rather refers to the deliberate choice to administer treatment to a patient based on one or more predetermined criteria.

As used herein, the term "predicting" indicates that the methods described herein provide information to enable a health care provider to determine the likelihood that an individual having a disorder will have a more accurate diagnosis or prognosis or respond more favorably to treatment. It does not refer to an ability to predict response with 100% accuracy. The skilled artisan will understand that it refers to an increased probability.

As used herein, "likelihood and "likely" provide a description of how probable an event is to occur. It may be used interchangeably with "probability." Likelihood refers to a probability that is more than speculation but less than certainty. Thus, an event is likely if a reasonable person using common sense, training or experience concludes that, given the circumstances, the event is probable. In some embodiments, once likelihood has been ascertained, the patient may be prognosed, diagnosed, treated or the treatment may be altered.

As used herein, the term "pharmaceutically acceptable" means a nontoxic material that does not interfere with the effectiveness of the biological activity of the active ingredient(s).

As used herein, the term "administering" in relation to a compound, e.g., serelaxin or a serelaxin receptor agonist, is used to refer to delivery of that compound to a patient by any route.

As used herein, "therapeutically effective amount" refers to an amount of serelaxin or a serelaxin agonist that is effective, upon single or multiple dose administration to a patient (such as a human) for treating, preventing, preventing the onset of, curing, delaying, reducing the severity of, ameliorating at least one symptom of a disorder or recurring disorder, or prolonging the survival of the patient beyond that expected in the absence of such treatment. When applied to an individual active ingredient (e.g., serelaxin), administered alone, the term refers to that ingredient alone. When applied to a combination, the term refers to combined amounts of the active ingredients that result in the therapeutic effect, whether administered in combination, serially or simultaneously.

As used herein, the terms "treatment" and "treat" refer to both prophylactic or preventative treatment (as the case may be) as well as curative or disease modifying treatment, including treatment of a patient at risk of contracting the disease or suspected to have contracted the disease as well as patients who are ill or have been diagnosed as suffering from a disease or medical condition, and includes suppression of clinical relapse or exacerbation. The treatment may be administered to a patient having a medical disorder or who ultimately may acquire the disorder, in order to prevent, cure, delay the onset of, reduce the severity of, or ameliorate one or more symptoms of a disorder or recurring disorder, or in order to prolong the survival of a patient beyond that expected in the absence of such treatment.

As used herein, the singular forms "a," "an," and "the" include plural referents unless the content clearly dictates otherwise. Thus, for example, reference to "a protein" includes a mixture of two or more proteins, and reference to "the agent" includes reference to one or more agents and equivalents thereof known to those skilled in the art, and so forth.

It will be clear that the invention may be practiced otherwise than as particularly described in the foregoing description and examples. Numerous modifications and variations of the present invention are possible in light of the above teachings and, therefore, are within the scope of the appended claims.

It is to be understood that both the foregoing general description and the following detailed description are exemplary and explanatory only and are not restrictive of the invention, as claimed. Moreover, it must be understood that the invention is not limited to the particular embodiments described, as such may, of course, vary. Further, the terminology used to describe particular embodiments is not intended to be limiting, since the scope of the present invention will be limited only by its claim.

Unless defined otherwise, the meanings of all technical and scientific terms used herein are those commonly understood by one of ordinary skill in the art to which this invention belongs. One of ordinary skill in the art will also appreciate that any methods and materials similar or equivalent to those described herein can also be used to practice or test the invention.

Further, all numbers expressing quantities of ingredients, reaction conditions, % purity, polypeptide lengths, and so forth, used in the specification and claims, are modified by the term "about," unless otherwise indicated. Accordingly, the numerical parameters set forth herein are approximations that may vary depending upon the desired properties of the present invention. At the very least, and not as an attempt to limit the application of the doctrine of equivalents to the scope of the claims, each numerical parameter should at least be construed in light of the number of reported significant digits, applying ordinary rounding techniques.

### Techniques for Assaying, Diagnostic Methods and Methods of Producing a Transmittable Form of Information

The claimed invention generally relates to serelaxin for use in the treatmentof patients with acute hear failure, as well as serelaxin for use in predicting the likelihood of such a patient's response to treatment with serelaxin. These methods employ, *inter alia,* determining the level of GDF-15 as a biomarker in a sample from the patient.

A biological sample from the patient may be assayed for the presence of GDF-15 by any applicable conventional means, which will be selected depending on the ease of acquiring a particular biological sample.

Numerous biological samples may be used to identify the presence of GDF-15, e.g., blood, synovial fluid, buffy coat, serum, plasma, lymph, feces, urine, tear, saliva, cerebrospinal fluid, buccal swabs, sputum, or tissue. Preferably, the biological sample comprises blood taken from a patient soon after experiencing a cardiac episode or soon after experiencing symptoms associated with a cardiac episode.

One inventive discovery of the present invention encompasses the determination that GDF-15 is useful for predicting the pulmonary load of a particular individual, which allows for a medical professional to proactively treat a potential cardiac episode such as acute heart failure.

Typically, once the presence of an AIR marker or polymorphism is determined, physicians or patients or other researchers may be informed of the result. Specifically the result can be cast in a transmittable form of information that can be communicated or transmitted to other researchers or physicians or patients. Such a form can vary and can be tangible or intangible. The result in the individual tested can be embodied in descriptive statements, diagrams, photographs, charts, images or any other visual forms. For example, statements regarding the level of GDF-15 are useful in indicating the testing results. These statements and visual forms can be recorded on a tangible media such as papers, computer readable media such as floppy disks, compact disks, etc., or on an intangible media, e.g., an electronic media in the form of email or website on internet or intranet.

In addition, the result can also be recorded in a sound form and transmitted through any suitable media, e.g., analog or digital cable lines, fiber optic cables, etc., via telephone, facsimile, wireless mobile phone, internet phone and the like. All such forms (tangible and intangible) would constitute a "transmittable form of information". Thus, the information and data on a test result can be produced anywhere in the world and transmitted to a different location.

### Serelaxin for use

The present invention provides serelaxin for use in selectively reducing the pulmonary load of a patient with acute heart failure (AHF) comprising:
obtaining a biological sample from a patient;
measuring the amount of GDF-15 in the biological sample; and
thereafter administering serelaxin in response to an elevated level of GDF-15, wherein serelaxin is administered subcutaneously at an infusion rate of 3 µg/kg/day to 150 µg/kg/day to maintain a serum concentration of 1 ng/ml to 100 ng/ml in the patient.

The present invention also provides serelaxin for use in a method of determining the prognosis of mortality of a patient with acute heart failure (AHF) comprising
obtaining a first biological sample from a patient;
detecting the level of GDF-15 in the first biological sample;
administering a therapeutically effective amount of serelaxin, wherein serelaxin is administered subcutaneously at an infusion rate of 3 µg/kg/day to 150 µg/kg/day to maintain a serum concentration of 1 ng/ml to 100 ng/ml in the patient;
obtaining a second biological sample from the patient after administration of serelaxin;
detecting the level of GDF-15 in the second biological sample;
comparing the level of GDF-15 in the first biological sample to the second biological sample; and
predicting the probability of survival, wherein a reduced level of GDF-15 in the second biological sample predicts an increased probability of survival.

The disclosed invention provides serelaxin for use in a personalized therapy for treating pulmonary load in patients with acute heart failure, i.e., they allow determination of whether to selectively treat the patient with a composition such as serelaxin. In this way, one can maximize the benefit and minimize the risk of a potentially fatal cardiac episode.

Serelaxin is to be administered via a subcutaneous pump supplying pharmaceutically active serelaxin (*e.g.,* synthetic, recombinant, analog, agonist, *etc*.) in an amount in a range of about 1 to 1000 µg/kg of subject body weight per day. In one embodiment, the dosages of serelaxin are 10, 30, 100 and 250 µg/kg/day. These dosages result in serum concentrations of serelaxin of about 1, 3, 10, 30, 75 or 100 ng/ml. In one preferred embodiment, pharmaceutically effective serelaxin or an agonist thereof is administered at about 30 µg/kg/day. In another preferred embodiment, pharmaceutically effective serelaxin or an agonist thereof is administered at about 10 to about 250 µg/kg/day. The administration of serelaxin is continued as to maintain a serum concentration of serelaxin of from from about 1 to about 100 ng/ml. Most preferably, the administration of serelaxin is continued as to maintain a serum concentration of serelaxin of 10 ng/ml or greater. These serelaxin concentrations can ameliorate or reduce the excessive dilute urine production and accompanying complication associated with NDI.

### Serelaxin Compositions and Formulations

Serelaxin is formulated as pharmaceutical to be used in the present invention. Any composition or compound that can stimulate a biological response associated with the binding of biologically or pharmaceutically active serelaxin (*e.g.,* synthetic serelaxin, recombinant serelaxin) can be used as a pharmaceutical in the disclosure. General details on techniques for formulation and administration are well described in the scientific literature (see Remington's Pharmaceutical Sciences, Maack Publishing Co, Easton Pa.). Pharmaceutical formulations containing pharmaceutically active serelaxin can be prepared according to any method known in the art for the manufacture of pharmaceuticals. The formulations containing pharmaceutically active serelaxin used in the invention can be formulated for subcutaneous administration in any conventionally acceptable way. Illustrative examples are set forth below. In one preferred embodiment, serelaxin is administered subcutaneously.

When serelaxin is delivered by subcutaneous injection (*e.g.,* infusion, bolus, pump), the formulations containing pharmaceutically active serelaxin can be in the form of a sterile injectable preparation, such as a sterile injectable aqueous or oleaginous suspension. This suspension can be formulated according to the known art using those suitable dispersing or wetting agents and suspending agents which have been mentioned above. The sterile injectable preparation can also be a sterile injectable solution or suspension in a nontoxic parenterally-acceptable diluent or solvent. Among the acceptable vehicles and solvents that can be employed are water and Ringer's solution, an isotonic sodium chloride. In addition, sterile fixed oils can conventionally be employed as a solvent or suspending medium. For this purpose any bland fixed oil can be employed including synthetic mono- or diglycerides. In addition, fatty acids such as oleic acid can likewise be used in the preparation of injectables.

Aqueous suspensions of the disclosure contain serelaxin in admixture with excipients suitable for the manufacture of aqueous suspensions. Such excipients include a suspending agent, such as sodium carboxymethylcellulose, methylcellulose, hydroxypropylmethylcellulose, sodium alginate, polyvinylpyrrolidone, gum tragacanth and gum acacia, and dispersing or wetting agents such as a naturally occurring phosphatide (*e.g.,* lecithin), a condensation product of an alkylene oxide with a fatty acid (*e.g.,* polyoxyethylene stearate), a condensation product of ethylene oxide with a long chain aliphatic alcohol (*e.g.,* heptadecaethylene oxycetanol), a condensation product of ethylene oxide with a partial ester derived from a fatty acid and a hexitol (*e.g.,* polyoxyethylene sorbitol mono-oleate), or a condensation product of ethylene oxide with a partial ester derived from fatty acid and a hexitol anhydride (*e.g.,* polyoxyethylene sorbitan monooleate). The aqueous suspension can also contain one or more preservatives such as ethyl or n-propyl p-hydroxybenzoate, one or more coloring agents, one or more flavoring agents and one or more sweetening agents, such as sucrose, aspartame or saccharin. Formulations can be adjusted for osmolarity.

Oil suspensions can be formulated by suspending serelaxin in a vegetable oil, such as arachis oil, olive oil, sesame oil or coconut oil, or in a mineral oil such as liquid paraffin. The oil suspensions can contain a thickening agent, such as beeswax, hard paraffin or cetyl alcohol. Sweetening agents can be added to provide a palatable oral preparation. These formulations can be preserved by the addition of an antioxidant such as ascorbic acid.

Dispersible powders and granules of the disclosure suitable for preparation of an aqueous suspension by the addition of water can be formulated from serelaxin in admixture with a dispersing, suspending and/or wetting agent, and one or more preservatives. Suitable dispersing or wetting agents and suspending agents are exemplified by those disclosed above. Additional excipients, for example sweetening, flavoring and coloring agents, can also be present.

The pharmaceutical formulations of the disclosure can also be in the form of oil-in-water emulsions. The oily phase can be a vegetable oil, such as olive oil or arachis oil, a mineral oil, such as liquid paraffin, or a mixture of these. Suitable emulsifying agents include naturally-occurring gums, such as gum acacia and gum tragacanth, naturally occurring phosphatides, such as soybean lecithin, esters or partial esters derived from fatty acids and hexitol anhydrides, such as sorbitan mono-oleate, and condensation products of these partial esters with ethylene oxide, such as polyoxyethylene sorbitan mono-oleate.

### Administration and Dosing Regimen of Serelaxin Formulations

The formulations containing pharmaceutically active serelaxin for use according to the invention are administered subcutaneously. Administration will vary with the pharmacokinetics and other properties of the drugs and the patients' condition of health. General guidelines are presented below.

The state of the art allows to determine the dosage regimen of serelaxin for each individual patient. As an illustrative example, the guidelines provided below for serelaxin can be used as guidance to determine the dosage regimen, *i.e.,* dose schedule and dosage levels, of formulations containing pharmaceutically active serelaxin when administered according to the invention. As a general guideline, it is expected that the daily dose of pharmaceutically active H1, H2 and/or H3 human serelaxin (*e.g.,* synthetic, recombinant, analog, agonist, *etc*.) is typically in an amount in a range of about 1 to 1000 µg/kg of subject body weight per day. In one embodiment, the dosages of serelaxin are 10, 30, 100 and 250 µg/kg/day. In another embodiment, these dosages result in serum concentrations of serelaxin of about 1, 3, 10, 30, 75 or 100 ng/ml. In one preferred embodiment, pharmaceutically effective serelaxin for use according to the invention is to be administered at about 30 µg/kg/day. In another preferred embodiment, pharmaceutically effective serelaxin for use according to the invention is to be administered at about 10 to about 250 µg/kg/day. In another embodiment, the administration of serelaxin is to be continued as to maintain a serum concentration of serelaxin of from about 1 to about 100 ng/ml. Most preferably, the administration of serelaxin is to be continued as to maintain a serum concentration of serelaxin of 10 ng/ml or greater. Thus, the present invention includes serelaxin for use in administrations that result in these serum concentrations of serelaxin. These serelaxin concentrations can ameliorate or reduce fluid accumulation associated with edema, including, but not limited to cerebral edema, ocular edema, pulmonary edema, ascites, hereditary angioedema, peripheral edema, and systemic edema. Furthermore, these serelaxin concentrations can ameliorate or reduce chronic excretion of dilute urine in NDI. Depending on the subject, the serelaxin administration is to be maintained for as specific period of time or for as long as needed to achieve stability in the subject. For example, the duration of serelaxin administration is preferably to be kept at a range of about 4 hours to about 96 hours, more preferably 8 hours to about 72 hours, depending on the patient, and one or more optional repeat treatments as needed.

Single or multiple administrations of serelaxin formulations may be administered depending on the dosage and frequency as required and tolerated by the patient who suffers from edema and/or NDI. The formulations should provide a sufficient quantity of serelaxin to effectively ameliorate the condition. A typical pharmaceutical formulation for subcutaneous administration of serelaxin would depend on the specific use. For example, serelaxin may be administered to a patient through monotherapy (*i.e.,* with no other concomitant medications) or in combination therapy with another medication. In one embodiment, serelaxin is to be administered to a patient daily as monotherapy. In another embodiment, serelaxin is to be administered to a patient daily as combination therapy with another drug. Notably, the dosages and frequencies of serelaxin administered to a patient may vary depending on age, degree of illness, drug tolerance, and concomitant medications and conditions.

The details of one or more embodiments of the disclosure are set forth in the accompanying description above. Although any methods and materials similar or equivalent to those described herein can be used in the practice or testing of the present disclosure, the preferred methods and materials are now described. Other features, objects, and advantages of the disclosure will be apparent from the description and from the claims. In the specification and the appended claims, the singular forms include plural referents unless the context clearly dictates otherwise. Unless defined otherwise, all technical and scientific terms used herein have the same meaning as commonly understood by one of ordinary skill in the art to which this disclosure belongs. All numerical ranges in this disclosure are inclusive of the endpoints and of all integers, decimals and fractions therebetween whether specifically stated or not. The following Examples are presented in order to more fully illustrate the preferred embodiments of the disclosure. These examples should in no way be construed as limiting the scope of the disclosed patent matter, as defined by the appended claims.

### Example 1: Effect of Serelaxin on GDF-15 Levels in Patients with Acute Heart Failure

In an international, double-blind, placebo-controlled trial (Teerlink et al., Lancet 381:29-39 (2013)) the entire contents of which are herein incorporated by reference, patients displaying pulmonary congestion on chest radiograph and admitted to the hospital for acute heart failure were randomized to a 48 hour intravenous infusion of placebo or 30 ug/kg/d serelaxin. Time elapsed from arrival to the hospital to the intravenous administration of serelaxin was less than nine hours. Serelaxin significantly improved the primary dyspnea efficacy endpoint as evaluated by the Visual Acuity Scale area under the curve (p=0.007) and patients reported improvements in general well-being. The average length of hospital stay was significantly reduced in the serelaxin treated group by 0.9 days (p=0.04) and time in the intensive care or coronary care unit was reduced by 0.4 days (p=0.03). The 48 hour infusion of serelaxin reduced cardiovascular death at 180 days (p=0.28). No previous intervention outcome trial in patients with acute heart failure has shown a beneficial effect on post discharge mortality.

Blood samples for biomarker analysis were collected in serum separating tubes prior to initiation of serelaxin or placebo (baseline) and at days 2, 5, 14, and 60. The serum samples were centrifuged within 30 to 60 minutes of collection following visualization of a clot, frozen at -20°C for up to four weeks, then sent on dry ice to a central laboratory for storage at -80°C until the analysis was performed. GDF-15 was measured in the samples using a pre-commercial electrochemiluminescent immunoassay provided by Roche Diagnostics GmbH (Mannheim, Germany). All samples from the same patient were analyzed at the same time at a certified central laboratory by personnel blinded to patient treatment and study data. The reporting range for GDF-15 was 400 to 20,000 ng/L.

Changes in patient-reported dyspnea were assessed through day 5 as the area under the change from baseline in visual analog scale score through day 5, where the worst possible score was assigned following death or worsening heart failure (dyspnea VAS AUC). Reasons for rehospitalizations through day 60 and causes of deaths through day 180 were adjudicated centrally by a blinded endpoint committee.

Multivariable linear regression models were developed for the changes in GDF-15 levels from baseline to days 2 and 14 using baseline patient clinical characteristics and routine laboratory measures; backwards elimination in the placebo group was used, with p<0.10 as the criterion for retention in the model. Missing predictors were imputed with the treatment-group-specific median for continuous variables and mode for categorical variables. GDF-15 values were log-transformed. The linearity of associations was assessed using restricted cubic splines, and if significant non-linearity was found (at p<0.10), a dichotomized, trichotomized, linear spline or quadratic or cubic polynomial transformation was chosen based on the univariable Akaike's Information Criterion. Adjusted R² values from 5-fold cross-validations are presented. The serelaxin effect on biomarker changes was then estimated in all patients with multivariable adjustment for covariates prognostic of these changes in the placebo group.

**TABLE 1. Serelaxin lowers GDF-15 in patients with acute heart failure**

| | | | | | **GeoMean Change vs Baseline** | | |
|---|---|---|---|---|---|---|---|
| **GDF-15 (ng/l)** | **Median Placebo (n=546)** | **Median Serelaxin (n=530)** | **GeoMean Placebo** | **GeoMean Serelaxin** | **Placebo** | **Serelaxin** | **Between Treatment** |
| Baseline | 3998.5 | 4115.5 | 4273.3 | 4380.3 | | | |
| Day 2 | 3617.0 | 3341.0 | 3855.6 | 3395.6 | -9.6%* | -21.6%* | <0.0001 |
| Day 5 | 3616.0 | 3584.5 | 3902.8 | 3690.3 | -8.3%* | -14.95* | 0.0244 |
| Day 14 | 3505.0 | 3296.0 | 3622.9 | 3397.0 | 15.7%* | -20.2%* | 0.0534 |
| Day 60 | 3092.0 | 2981.5 | 3208.7 | 3114.0 | 22.5%* | -26.4%* | 0.3255 |

| | | | | | | | |
|---|---|---|---|---|---|---|---|
| * P>0.05 vs baseline using GeoMean 95% confidence intervals | | | | | | | |

At days 2 and 5, GDF-15 levels were significantly lower in acute heart failure patients treated with serelaxin in addition to the standard of care than in those treated with placebo in addition to standard of care. At day 14, GDF-15 levels approached statistical significance.

Factors affecting change in GDF-15 levels from baseline to day 2 of serelaxin treatment are shown in Table 2. Increases in GDF-15 were associated with older age, peripheral vascular disease, aortic stenosis and lower NT-pro-BNP at baseline.

**TABLE 2. Factors affecting GDF-15 levels at day 2 of serelaxin treatment**

| **Characteristic** | **Mean Change (95% CI)** | **p-value** |
|---|---|---|
| Age (y) | 0.022 (0.012,0.031) | 0.000 |
| Male | -0.106 (-0.322,0.110) | 0.334 |
| US-like | -0.082 (-0.305,0.141) | 0.469 |
| Most recent ejection fraction (%) | 0.007 (-0.001,0.014) | 0.086 |
| CHF 1 month prior | -0.123 (-0.359,0.113) | 0.305 |
| NYHA class 30 days before | . | 0.264 |
| . | -0.224 (-0.514,0.065) | |
| . | 0.012 (-0.252,0.276) | |
| . | -0.194 (-0.547,0.158) | |
| Weight (kg) | 0 (-0.006,0.006) | 0.947 |
| Height (cm) | -0.009 (-0.021,0.002) | 0.095 |
| Body mass index (kg/m²) | 0.005 (-0.012,0.023) | 0.550 |
| Systolic BP (mmHg) | 0.003 (-0.003,0.009) | 0.317 |
| Diastolic BP (mm H)g | 0 (-0.008,0.008) | 0.970 |
| Pulse pressure (mmHg) | 0.003 (-0.003,0.009) | 0.339 |
| Heart rate (beats/min) | 0.006 (-0.001,0.013) | 0.087 |
| Respiratory rate (breaths/min) | 0.018 (-0.005,0.041) | 0.119 |
| Body temperature (°C) | -0.125 (-0.404,0.155) | 0.382 |
| HF hospitalization past year | -0.172 (-0.400,0.056) | 0.140 |
| Number of HF hospitalizations past year | -0.103 (-0.221,0.014) | 0.084 |
| Edema (0-3) | . | 0.077 |
| . | -0.263 (-0.564,0.037) | |
| . | -0.296 (-0.597,0.005) | |
| . | 0.008 (-0.322,0.337) | |
| Orthopnea (0-3) | . | 0.666 |
| . | 0.239 (-0.329,0.806) | |
| . | 0.209 (-0.332,0.749) | |
| . | 0.308 (-0.234,0.849) | |
| Orthopnea (0-3) (ordinal) | 0.063 (-0.061,0.187) | 0.318 |
| Rales (0-3) | . | 0.015 |
| . | 0.479 (-0.003,0.961) | |
| . | 0.694 (0.215,1.173) | |
| . | 0.731 (0.138,1.324) | |
| Jugular venous pressure (0-2) | . | 0.289 |
| . | 0.054 (-0.207,0.315) | |
| . | -0.144 (-0.427,0.140) | |
| Dyspnea on exertion (0-3) | . | 0.227 |
| . | -1.085 (-2.888,0.719) | |
| . | -1.224 (-2.973,0.525) | |
| . | -1.022 (-2.765,0.721) | |
| Dyspnea by VAS (mm) | 0.005 (-0.001,0.010) | 0.086 |
| Hyperlipidemia | -0.235 (-0.445,-0.025) | 0.028 |
| Diabetes mellitus | -0.074 (-0.284,0.137) | 0.492 |
| Hypertension | -0.193 (-0.512,0.126) | 0.236 |
| Stroke or other cerebrovascular event | -0.171 (-0.465,0.122) | 0.253 |
| Asthma bronchitis or COPD | 0.047 (-0.247,0.341) | 0.753 |
| Ischemic heart disease | -0.118 (-0.329,0.093) | 0.272 |
| Myocardial infarction | 0.011 (-0.209,0.231) | 0.923 |
| Coronary artery bypass graft | -0.199 (-0.461,0.064) | 0.138 |
| Percutaneous intervention | -0.146 (-0.390,0.097) | 0.240 |
| Angina | 0.008 (-0.322,0.339) | 0.960 |
| CCS Class (I/II/III/IV) | . | 0.562 |
| . | 0.066 (-0.354,0.485) | |
| . | 0.06 (-0.951,1.072) | |
| . | -1.749 (-4.214,0.717) | |
| Peripheral vascular disease | -0.001 (-0.303,0.300) | 0.993 |
| Substance abuse | -0.902 (-1.566,-0.238) | 0.008 |
| Cigarette smoking | -0.451 (-0.764,-0.138) | 0.005 |
| Depression | 0.295 (-0.168,0.758) | 0.211 |
| Hypothyroid | -0.173 (-0.583,0.237) | 0.407 |
| Hyperthyroid | -0.324 (-0.900,0.251) | 0.270 |
| Malignancy | -0.187 (-0.607,0.233) | 0.382 |
| Mitral stenosis | -0.218 (-1.231,0.795) | 0.673 |
| Mitral regurgitation | -0.093 (-0.319,0.133) | 0.421 |
| Aortic stenosis | -0.242 (-0.757,0.273) | 0.357 |
| Aortic regurgitation | 0.526 (0.013,1.039) | 0.044 |
| History of atrial fibrillation or flutter | -0.046 (-0.256,0.165) | 0.672 |
| Atrial fibrillation/flutter at screening | 0.09 (-0.122,0.303) | 0.405 |
| Hemoglobin (g/dL) | 0.069 (0.010,0.128) | 0.023 |
| Hematocrit (%) | 0.02 (0.001,0.040) | 0.038 |
| Creatinine (umol/L) | 0 (-0.003,0.003) | 0.934 |
| BUN (mmol/L) | -0.015 (-0.041,0.011) | 0.271 |
| eGFR | 0 (-0.007,0.006) | 0.886 |
| Uric acid (umol/L) | 0 (-0.001,0.000) | 0.366 |
| Total bilirubin (umol/L) | -0.006 (-0.018,0.005) | 0.254 |
| Alanine aminotransferase (U/L) | -0.032 (-0.153,0.090) | 0.611 |
| Aspartate aminotransferase U/L | 0.107 (-0.051,0.265) | 0.183 |
| Alkaline phosphatase (U/L) | -0.002 (-0.004,0.001) | 0.175 |
| Phosphate (mmol/L) | 0.137 (-0.363,0.637) | 0.591 |
| Sodium (mmol/L) | 0.001 (-0.028,0.031) | 0.931 |
| Potassium (mmol/L) | -0.058 (-0.245,0.128) | 0.540 |
| Calcium (mmol/L) | -0.261 (-0.962,0.439) | 0.465 |
| Total cholesterol (mmol/L) | 0.093 (0.003,0.182) | 0.043 |
| Glucose (mmol/L) | 0.024 (-0.002,0.050) | 0.073 |
| Albumin (g/L) | 0 (-0.024,0.024) | 0.989 |
| Total protein (g/L) | -0.007 (-0.025,0.010) | 0.424 |
| Troponin T (ug/L) | 0.137 (0.051,0.223) | 0.002 |
| NT-proBNP (ng/L) | -0.036 (-0.116,0.044) | 0.374 |

Changes in GDF-15 at day 2 and day 14 were associated with adverse outcomes in acute heart failure patients. Hospital readmission for heart failure or renal failure, cardiovascular death at day 60 and cardiovascular death at day 180 were strongly and independently associated with the change observed in GDF-15 levels at days 2 and 14 post-serelaxin treatment, as shown in Table 3. Increases in GDF-15 from baseline to day 2 and to day 14 were highly significant predictors of these negative outcomes.

**TABLE 3. Association of GDF-15 with negative outcomes in acute heart failure patients**

| **Dyspnea** | | | | | | | | |
|---|---|---|---|---|---|---|---|---|
| **Biomarker** | **Unadjusted** | | **Multivariable adjusted without serelaxin** | | **Multivariable adjusted without serelaxin** | | **Multivariable biomarker adjusted without serelaxin** | |
| GDF-15 | **Mean Change** | **p-value** | **Mean Change** | **p-value** | **Mean Change** | **p-value** | **Mean Change** | **p-value** |
| Baseline (n= 1088) | -200.0 (-375.7,-24.33) | 0.026 | -79.85 (-236.4, 76.720) | 0.318 | -84.67 (-240.7, 71.381) | 0.288 | -88.91 (-246.1, 68.324) | 0.268 |
| Change at day 2 (ratio of D2 to BL) (n= 1073) | | 0.000 | | 0.002 | | 0.005 | | 0.193 |
| | -222.0 (-316.5,-127.5) | . | -135.0 (-212.9,-56.96) | . | -123.0 (-201.5,-44.42) | . | -59.36 (-146.6,27.868) | . |

| **Rehospitalization or cardiovascular death through day 60** | | | | | | | | |
|---|---|---|---|---|---|---|---|---|
| **Biomarker** | **Unadjusted** | | **Multivariable adjusted without serelaxin** | | **Multivariable adjusted without serelaxin** | | **Multivariable biomarker adjusted without serelaxin** | |
| **GDF-15** | **HR (95% CI)** | **p-value** | **HR (95% CI)** | **p-value** | **HR (95% CI)** | **p-value** | **HR (95% CI)** | **p-value** |
| | 1.341 (1.143,1.573) | 0.000 | 1.076 (0.901,1.285) | 0.421 | 1.076 (0.901,1.285) | 0.420 | 1.083 (0.904,1.296) | 0.388 |
| | 1.473 (1.150,1.887) | 0.002 | 1.493 (1.147,1.942) | 0.003 | 1.505 (1.154,1.963) | 0.003 | 1.442 (1.068,1.947) | 0.017 |
| | 1.879 (1.471,2.401) | 0.000 | 1.722 (1.346,2.204) | 0.000 | 1.727 (1.349,2.212) | 0.000 | 1.675 (1.299,2.160) | 0.000 |

| **Cardiovascular death through day 180** | | | | | | | | |
|---|---|---|---|---|---|---|---|---|
| **Biomarker** | **Unadjusted** | | **Multivariable adjusted without serelaxin** | | **Multivariable adjusted without serelaxin** | | **Multivariable biomarker adjusted without serelaxin** | |
| | **HR (95% CI)** | **p-value** | **HR (95% CI)** | **p-value** | **HR (95% CI)** | **p-value** | **HR (95% CI)** | **p-value** |
| | 1.597 (1.311,1.945) | 0.000 | 1.120 (0.883,1.422) | 0.350 | 1.121 (0.885,1.422) | 0.344 | 1.143 (0.897,1.458) | 0.280 |
| | 1.493 (1.090,2.044) | 0.012 | 1.475 (1.037,2.100) | 0.031 | 1.414 (0.989, 2.021) | 0.058 | 1.195 (0.805,1.774) | 0.376 |
| | 1.986 (1.469,2.685) | 0.000 | 1.953 (1.395,2.734) | 0.000 | 1.943 (1.381,2.734) | 0.000 | 1.876 (1.323,2.661) | 0.000 |

A multivariate analysis of the data demonstrated that the baseline level of GDF-15 was not associated with increased rates of hospital readmission for heart failure or renal failure; nor was it associated with cardiovascular death through day 60 or day 180. The changes in GDF-15 from baseline to day 2 and to day 14 were highly significant predictors of these outcomes. Serelaxin treatment was associated with a faster decrease in GDF-15 to day 14 and a trend towards lower GDF-15 at day 60. A multivariate analysis of 180 day cardiovascular mortality that incorporates both serelaxin treatment and changes in GDF-15 to day 2, as shown in Table 4, demonstrate that the decrease in GSF-15 is partially responsible for the previous observation that serelaxin treatment decreased mortality in patients with acute heart failure. Inclusion of the GDF-15 change to day 14 in a multivariable model of 180-day CV mortality reduced the estimated serelaxin hazard ratio to 0.753, while the effect of the GDF-15 change remained highly significant, suggesting that the change in GDF-15 may mediate the association of serelaxin treatment with decreased 180-day mortality.

**TABLE 4. Multivariate analysis of 180 day cardiovascular mortality**

| **Covariate** | **HR** | **95% CI** | **p value** |
|---|---|---|---|
| US-like | 0.465 | (0.293,0.738) | 0.001 |
| Systolic BP (mmHg) | 0.981 | (0.965,0.997) | 0.018 |
| Orthopnea (0-3), ordinal | 1.630 | (1.212,2.191) | 0.001 |
| Angina | 1.735 | (0.945,3.186) | 0.076 |
| Hyperthyroid | 2.222 | (0.877,5.632) | 0.092 |
| Mitral regurgitation | 0.749 | (0.459,1.221) | 0.246 |
| Atrial fibrillation/flutter at screening | 1.513 | (0.959,2.386) | 0.075 |
| BUN (mmol/L) | 1.089 | (1.041,1.139) | 0.000 |
| Sodium (mmol/L) | 0.928 | (0.882,0.977) | 0.004 |
| Potassium (mmol/L) | 1.241 | (0.892,1.728) | 0.200 |
| Calcium (mmol/L) | 0.215 | (0.045,1.033) | 0.055 |
| Total protein (g/L) | 1.039 | (0.996,1.083) | 0.074 |
| Troponin T (ug/L) | 1.396 | (1.176,1.656) | 0.000 |
| NT-proBNP (ng/L) | 1.352 | (1.110,1.645) | 0.003 |
| Baseline GDF-15 | 1.180 | (0.900,1.546) | 0.231 |
| Change in GDF-15 (ratio of D2 to BL) | 1.414 | (0.989,2.021) | 0.058 |
| Serelaxin | 0.763 | (0.481,1.208) | 0.248 |

### Example 2: Effect of Serelaxin on GDF-15 and Pulmonary Hemodynamics in Patients with Acute Heart Failure

In a separate study, the pulmonary hemodynamic effects (e.g., the pulomonary load) of serelaxin were evaluated in patients hospitalized with acute heart failure (Ponikowski et al., Eur Heart J 35:431-441 (2014)). Patients were randomized 1:1 to serelaxin or placebo, initially stabilized, then infused with serelaxin at a dose of 30 ug/kg/d for 20 hours. Pulmonary congestion at the time of presentation was a required inclusion criterion. Time elapsed from hospital admission to the beginning of intravenous infusion of serelaxin was less than 29 hours. Serelaxin exerted rapid hemodynamic effects; changes were detected within the first 30 minutes of infusion and were sustained throughout the treatment period. Serelaxin reduced the time weighted average pulmonary capillary wedge pressure from baseline in the first eight hours of treatment (p=0.0001) as well as during hours 8-20 (p=0.03). Serelaxin significantly lowered both systolic and diastolic pulmonary arterial pressure (p=0001 at four hours) with a concomitant decrease in systemic vascular resistance and pulmonary vascular resistance.

GDF-15 was measured in blood samples collected in serum separating tubes. The serum samples were centrifuged within two hours and plasma was stored at -20°C or lower, followed by storage at -80°C until the analysis was performed. GDF-15 was measured using a pre-commercial electrochemiluminescent immunoassay provided by Roche Diagnostics GmbH (Mannheim, Germany). All samples from the same patient were analyzed at the same time at a certified central laboratory by personnel blinded to patient treatment and study data.

Pulmonary capillary wedge pressure (PCWP), mean pulmonary artery pressure (PAP), and pulmonary vascular resistance (PVR) were measured as well as NTproBNP and GDF-15. An analysis was performed of the association of GDF-15 and NTproBNP with these hemodynamic indices.

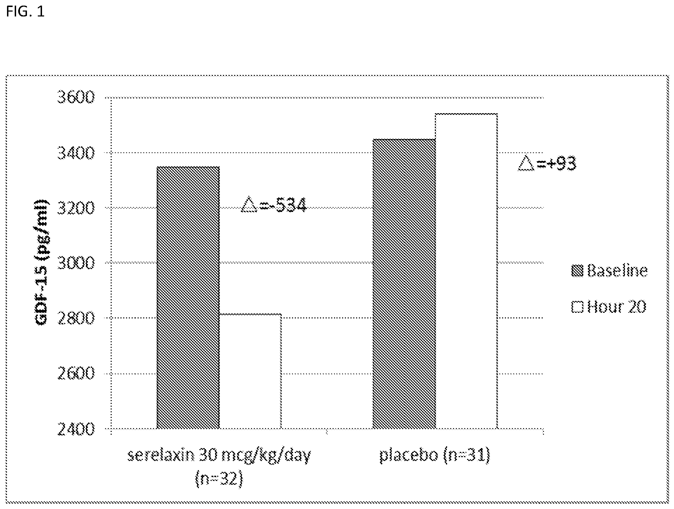

As with example 1, above, the GDF-15 levels were significantly decreased by serelaxin versus placebo (baseline median 3262 and 3192 ng/ml, respectively and shown in FIG. 1). As shown in FIG. 1, the geo-mean was reduced from baseline by 16%, while the placebo showed an increase of 3% from baseline. This resulted in a treatment difference of 18% in favor of serelaxin (p=0.0204). In addition, over the 20 hour infusion period, improvements in Pulmonary Capillary Wedge Pressure (PCWP), mean Pulmonary Artery Pressure (PAP), and Pulmonary Vascular Resistance (PVR) were measured as well as NTproBNP and GDF-15. An analysis was performed of the association of GDF-15 and NTproBNP with these hemodynamic indices.

Over the 20 hour infusion period, improvements in PCWP, PAP and PVR favored serelaxin compared to placebo (all p values <0.005). NTproBNP was also significantly reduced by serelaxin (p=0.037).

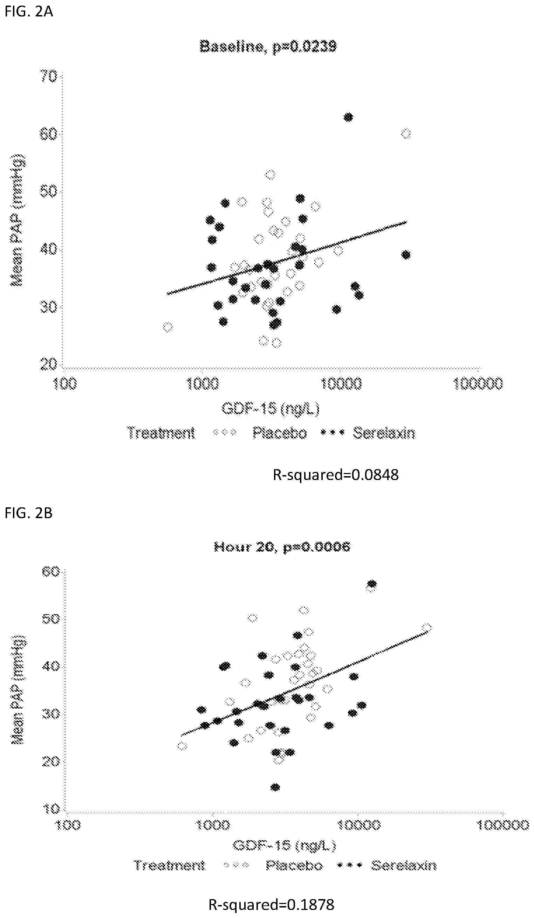

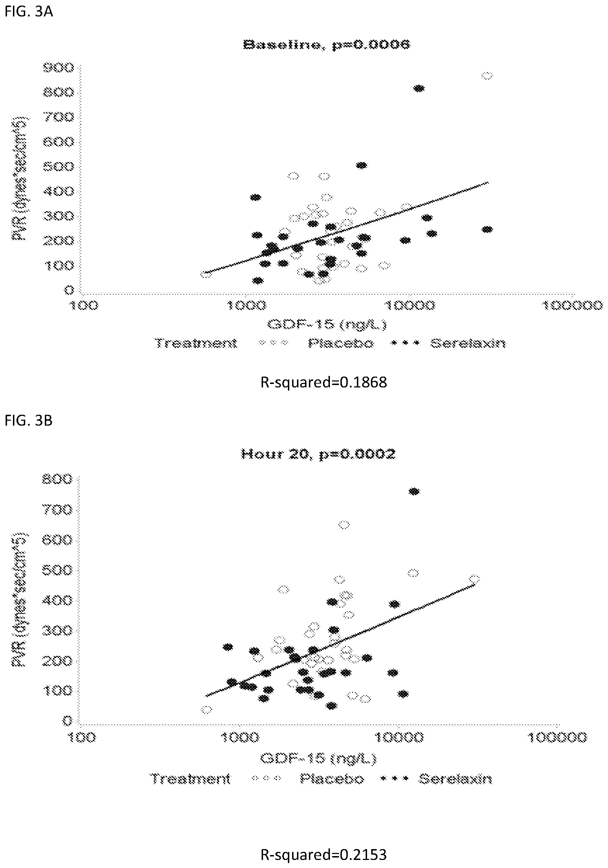

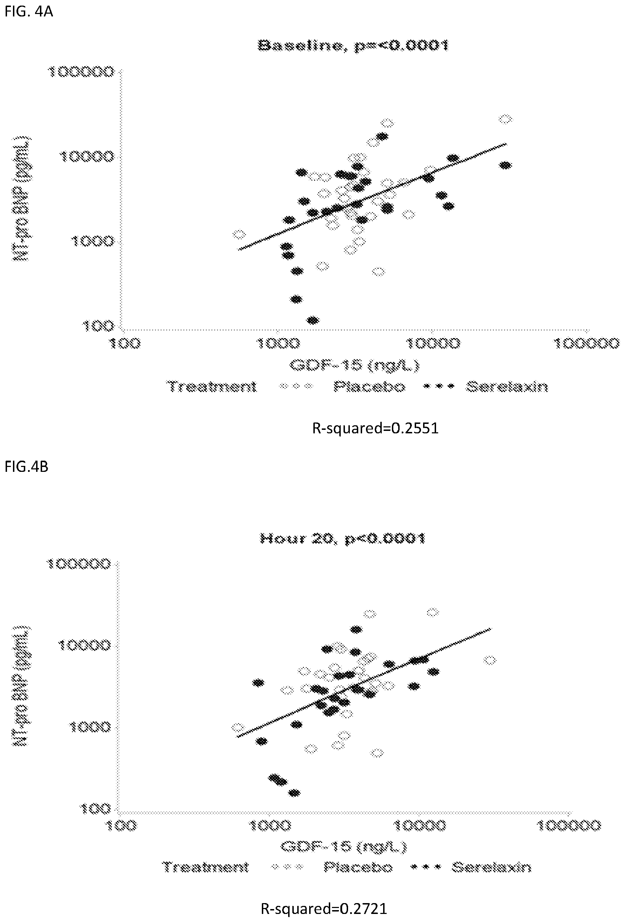

GDF-15 levels at baseline and 20 hours were significantly associated with PAP (FIGs. 2A-B, p<0.025), PVR (FIGS. 3A-B, p< 0.001) and NTproBNP (FIGS. 4A-B, p<0.001). GDF-15 levels were significantly associated with PCWP at 20 hours but not baseline (p<0.02; p=0.7). The association of NTproBNP levels at baseline and 20 hours was not statistically significant for PAP, PVR, or PCWP

This example shows that not only did serelaxin reduce GDF-15 levels, but that additionally serelaxin had favorable effects on pulmonary hemodynamics and significantly reduced NTproBNP and GDF-15. GDF-15 levels at baseline and 20 hours were significantly associated with NTproBNP and several pulmonary hemodynamic indices. In contrast, NTproBNP was not significantly associated with the pulmonary indices at baseline or 20 hours. Thus, GDF-15 at baseline as well as reductions in GDF-15 over time, are more useful than NTproBNP for predicting pulmonary load.

Overall then, the inventors have surprisingly discovered that the levels of GDF-15 at baseline, and for a period of up to 14 days thereafter can be used to predict a patient's pulmonary load. This in turn, allows for the prediction of a potential cardiac event, as it is well known that an increased pulmonary load can lead to a cardiac event. As a result, a healthcare provider can act accordingly and administer a GDF-15 lowering composition such as serelaxin.

### Example 3: Statistical Methods

Multivariable linear regression models were developed for the changes in biomarker levels from baseline to day 2 and 14 using baseline patient clinical characteristics and routine laboratory measures; backwards elimination in the placebo group was used, with p<0.10 as the criterion for retention in the model. Missing predictors were imputed with the treatment-group-specific median for continuous variables and mode for categorical variables. GDF-15 values were log-transformed. To allow consistency and comparability of models with and without biomarkers, missing follow-up biomarker levels were imputed using linear interpolation or as last observation carried forward if no following measure was available. The linearity of associations was assessed using restricted cubic splines, and if significant non-linearity was found (at p<0.10), a dichotomized, trichotomized, linear spline or quadratic or cubic polynomial transformation was chosen based on the univariable Akaike's Information Criterion. The serelaxin effect on GDF-15 changes was then estimated with multivariable adjustment for covariates prognostic of these changes in the placebo group. The results are set forth in Tables 5 and 6 below.

Associations of baseline biomarker values and changes with clinical outcomes were estimated with linear regression for dyspnea VAS AUC, and with Cox regression models for 60-day rehospitalization for heart failure or renal failure or cardiovascular death and for 180-day cardiovascular mortality. Linearity of associations of GDF-15 values with outcomes was assessed as above. Mean changes in biomarkers over time were estimated from repeated measures analysis of variance models, with no imputation for missing values.

**TABLE 5. Residual effect of serelaxin and effect of change in GDF-15 from baseline to Day 2 on CV death through Day 180**

| **Covariates** | **HR for a change of** | **HR** | **95% CI** | **p-value** |
|---|---|---|---|---|
| **Serelaxin and change in GDF-15 from baseline to Day 2** | | | | |
| Baseline GDF-15 | double | 1.720 | (1.369, 2.162) | <0.001 |
| Change in GDF-15 (ratio of D2 to BL) | double | 1.430 | (1.042, 1.964) | 0.027 |
| Serelaxin | | 0.832 | (0.664, 1.043) | 0.111 |
| | | | | |

| **Serelaxin, change in GDF-15 from baseline to Day 2, and multivariable predictors of outcome** | | | | |
|---|---|---|---|---|
| US-like | | 0.465 | (0.293,0.738) | 0.001 |
| Systolic BP, mmHg | 1 | 0.981 | (0.965,0.997) | 0.018 |
| Orthopnea (0-3), ordinal | 1 | 1.630 | (1.212,2.191) | 0.001 |
| Angina | | 1.735 | (0.945,3.186) | 0.076 |
| Hyperthyroid | | 2.222 | (0.877,5.632) | 0.092 |
| Mitral regurgitation | | 0.749 | (0.459,1.221) | 0.246 |
| Atrial fibrillation/flutter at screening | | 1.513 | (0.959,2.386) | 0.075 |
| BUN, mmol/L | 1 | 1.089 | (1.041,1.139) | <0.001 |
| Sodium, mmol/L | 1 | 0.928 | (0.882,0.977) | 0.004 |
| Potassium, mmol/L | 1 | 1.241 | (0.892,1.728) | 0.200 |
| Calcium, mmol/L | 1 | 0.215 | (0.045,1.033) | 0.055 |
| Total protein, g/L | 1 | 1.039 | (0.996,1.083) | 0.074 |
| Troponin T, ug/L | double | 1.396 | (1.176,1.656) | <0.001 |
| NT-proBNP, ng/L | double | 1.352 | (1.110,1.645) | 0.003 |
| Baseline GDF-15 | double | 1.180 | (0.900,1.546) | 0.231 |
| Change in GDF-15 (ratio of D2 to BL) | double | 1.414 | (0.989,2.021) | 0.058 |
| Serelaxin | | 0.763 | (0.481,1.208) | 0.248 |
| **C-statistic (95% CI)** | Overall Observed: 0.815 (0.773,0.857) | | | |
| | Cross-validated: 0.793 (0.700,0.885) | | | |

The change in GDF-15 to day 2 remained a significant predictor of 180-day CV mortality (P=0.027) when added to the effect of serelaxin, even after adjustment for baseline covariates (P=0.058). Unadjusted for other baseline covariates, serelaxin treatment was associated with a hazard ratio of 0.619 (95% CI 0.403-0.950) for 180-day CV mortality; adjustment for GDF-15 change to day 2 reduced the observed serelaxin effect (HR 0.832, 95% CI 0.664-1.043). Similar reductions were observed after multivariable adjustment for baseline covariates (HRs 0.665, 95% CI 0.430-1.028 and 0.763, 95% CI 0.481-1.208, respectively).

Similar results were observed for GDF-15 change to day 14 as set forth in Table 6 below. These results suggest that changes in GDF-15 mediate the association of serelaxin treatment with decreased 180-day mortality.

**TABLE 6. Residual effect of serelaxin and effect of change in GDF-15 from baseline to Day 14 on CV death through Day 180**

| **Covariate adjustment** | **HR for a change of** | **HR** | **95% CI** | **p-value** |
|---|---|---|---|---|
| **Serelaxin and change in GDF-15 from baseline to Day 14 [1]** | | | | |
| Baseline GDF-15 | double | 1.947 | (1.496, 2.533) | <0.001 |
| GDF-15 change at D14 (ratio of D14 to BL) | double | 1.960 | (1.448, 2.655) | <0.001 |
| Serelaxin | | 0.847 | (0.659, 1.087) | 0.192 |
| | | | | |

| **Serelaxin, change in GDF-15 from baseline to Day 14, and multivariable predictors of outcome [1]** | | | | |
|---|---|---|---|---|
| US-like | | 0.414 | (0.245,0.702) | 0.001 |
| systolic BP, mmHg | 1 | 0.980 | (0.963,0.998) | 0.029 |
| orthopnea (0-3), ordinal | 1 | 1.652 | (1.185,2.302) | 0.003 |
| Angina | | 1.587 | (0.768,3.281) | 0.213 |
| Hyperthyroid | | 1.996 | (0.608,6.550) | 0.254 |
| Mitral regurgitation | | 0.756 | (0.439,1.301) | 0.312 |
| atrial fibrillation/flutter at screening | | 1.346 | (0.805,2.250) | 0.257 |
| BUN, mmol/L | 1 | 1.108 | (1.056,1.162) | <0.001 |
| sodium, mmol/L | 1 | 0.941 | (0.886,0.999) | 0.045 |

| **Covariate adjustment** | **HR for a change of** | **HR** | **95% CI** | **p-value** |
|---|---|---|---|---|
| Potassium, mmol/L | 1 | 1.195 | (0.819,1.743) | 0.356 |
| Calcium, mmol/L | 1 | 0.348 | (0.050,2.408) | 0.285 |
| Total protein, g/L | 1 | 1.032 | (0.984,1.081) | 0.196 |
| Troponin T, ug/L | double | 1.419 | (1.169,1.723) | <0.001 |
| NT-proBNP, ng/L | double | 1.332 | (1.080,1.643) | 0.007 |
| baseline GDF-15 | double | 1.348 | (0.984,1.848) | 0.063 |
| GDF-15 change at D14 (ratio of D14 to BL) | double | 1.943 | (1.381,2.734) | <0.001 |
| Serelaxin | | 0.753 | (0.454,1.249) | 0.272 |
| **C-statistic (95% CI)** | Overall Observed: 0.835 (0.789,0.880) | | | |
| | Cross-validated: 0.779 (0.654,0.903) | | | |

| | | | | |
|---|---|---|---|---|
| [1] Patients who died or were censored on or before day 14 were excluded (N=1037) | | | | |

As shown above, serelaxin was associated with a greater decreases in GDF-15 that were statistically significant at days 2 and 5 with a trend for a significant difference at day 14 (p=0.0534), although this was reduced after adjustment for baseline characteristics. The effect of serelaxin on GDF-15 appears to be of special importance. Although GDF-15 levels have been reported to be decreased following LVAD implantation and cardiac unloading in end-stage HF, serelaxin appears to be the first drug therapy shown to reduce GDF-15 in patients with cardiovascular disease. Angiotensin receptor blocker therapy did not reduce GDF-15 in CHF and intensive statin therapy had no effect on GDF-15 in other clinical trials.

## Claims

1. Serelaxin for use in selectively reducing the pulmonary load of a patient with acute heart failure (AHF) comprising:
obtaining a biological sample from a patient;
measuring the amount of GDF-15 in the biological sample; and
thereafter administering serelaxin in response to an elevated level of GDF-15, wherein serelaxin is administered subcutaneously at an infusion rate of 3 µg/kg/day to 150 µg/kg/day to maintain a serum concentration of 1 ng/ml to 100 ng/ml in the patient.

2. Serelaxin for use according to claim 1, wherein the pulmonary load is defined as Pulmonary capillary wedge pressure (PCWP), mean pulmonary artery pressure (PAP), or pulmonary vascular resistance (PVR).

3. Serelaxin for use according to any of the previous claims, wherein serelaxin is administered to maintain a serum concentration of 10 ng/ml in the patient.

4. Serelaxin for use according to any of the previous claims, wherein serelaxin is administered subcutaneously at an infusion rate of 30 µg/kg/day.

5. Serelaxin for use according to any of the previous claims, wherein serelaxin is administered continuously for at least 24 hours.

6. Serelaxin for use in a method of determining the prognosis of mortality of a patient with acute heart failure (AHF) comprising:
obtaining a first biological sample from a patient;
detecting the level of GDF-15 in the first biological sample;
administering a therapeutically effective amount of serelaxin, wherein serelaxin is administered subcutaneously at an infusion rate of 3 µg/kg/day to 150 µg/kg/day to maintain a serum concentration of 1 ng/ml to 100 ng/ml in the patient;
obtaining a second biological sample from the patient after administration of serelaxin;
detecting the level of GDF-15 in the second biological sample;
comparing the level of GDF-15 in the first biological sample to the second biological sample; and
predicting the probability of survival, wherein a reduced level of GDF-15 in the second biological sample predicts an increased probability of survival.

7. Serelaxin for use according to claim 6, wherein the second biological sample is obtained 2 or 14 days after administration of serelaxin.

8. Serelaxin for use according to any of claims 6-7, wherein serelaxin is administered to maintain a serum concentration of 10 ng/ml in the patient.

9. Serelaxin for use according to any of claims 6-8, wherein serelaxin is administered subcutaneously at an infusion rate of 30 µg/kg/day.

10. Serelaxin for use according to any of claims 6-9, wherein serelaxin is administered continuously for at least 24 hours.

## Patentansprüche

1. Serelaxin zur Verwendung bei der selektiven Reduzierung der Lungenbelastung eines Patienten mit akuter Herzinsuffizienz (AHF), umfassend:
Erhalten einer biologischen Probe von einem Patienten;
Messen der GDF-15-Menge in der biologischen Probe; und
anschließendes Verabreichen von Serelaxin als Reaktion auf einen erhöhten GDF-15-Spiegel, wobei Serelaxin zur Aufrechterhaltung einer Serumkonzentration von 1 ng/ml bis 100 ng/ml im Patienten subkutan mit einer Infusionsrate von 3 µg/kg/Tag bis 150 µg/kg/Tag verabreicht wird.

2. Serelaxin zur Verwendung nach Anspruch 1, wobei die Lungenbelastung als Lungenkapillaren-Verschlussdruck (PCWP), mittlerer pulmoarterieller Druck (PAP) oder pulmonaler Gefäßwiderstand (PVR) definiert ist.

3. Serelaxin zur Verwendung nach einem der vorhergehenden Ansprüche, wobei Serelaxin zur Aufrechterhaltung einer Serumkonzentration von 10 ng/ml im Patienten verabreicht wird.

4. Serelaxin zur Verwendung nach einem der vorhergehenden Ansprüche, wobei Serelaxin subkutan mit einer Infusionsrate von 30 µg/kg/Tag verabreicht wird.

5. Serelaxin zur Verwendung nach einem der vorhergehenden Ansprüche, wobei Serelaxin kontinuierlich mindestens 24 Stunden verabreicht wird.

6. Serelaxin zur Verwendung in einem Verfahren zur Bestimmung der Mortalitätsprognose eines Patienten mit akuter Herzinsuffizienz (AHF), umfassend:
Erhalten einer ersten biologischen Probe von einem Patienten;
Nachweisen des GDF-15-Spiegels in der ersten biologischen Probe;
Verabreichen einer therapeutisch wirksamen Menge von Serelaxin, wobei Serelaxin zur Aufrechterhaltung einer Serumkonzentration von 1 ng/ml bis 100 ng/ml im Patienten subkutan mit einer Infusionsrate von 3 µg/kg/Tag bis 150 µg/kg/Tag verabreicht wird;
Erhalten einer zweiten biologischen Probe vom Patienten nach Verabreichung von Serelaxin;
Nachweisen des GDF-15-Spiegels in der zweiten biologischen Probe;
Vergleichen des GDF-15-Spiegels in der ersten biologischen Probe mit der zweiten biologischen Probe; und
Vorhersagen der Überlebenswahrscheinlichkeit, wobei ein reduzierter GDF-15-Spiegel in der zweiten biologischen Probe eine erhöhte Überlebenswahrscheinlichkeit vorhersagt.

7. Serelaxin zur Verwendung nach Anspruch 6, wobei die zweite biologische Probe 2 oder 14 Tage nach Verabreichung von Serelaxin erhalten wird.

8. Serelaxin zur Verwendung nach einem der Ansprüche 6-7, wobei Serelaxin zur Aufrechterhaltung einer Serumkonzentration von 10 ng/ml im Patienten verabreicht wird.

9. Serelaxin zur Verwendung nach einem der Ansprüche 6-8, wobei Serelaxin subkutan mit einer Infusionsrate von 30 µg/kg/Tag verabreicht wird.

10. Serelaxin zur Verwendung nach einem der Ansprüche 6-9, wobei Serelaxin kontinuierlich mindestens 24 Stunden verabreicht wird.

## Revendications

1. Sérélaxine pour une utilisation dans la réduction sélective de la charge pulmonaire d'un patient présentant une insuffisance cardiaque aiguë (ICA) comprenant :
l'obtention d'un échantillon biologique d'un patient ;
la mesure de la quantité de GDF-15 dans l'échantillon biologique ; et
puis l'administration de sérélaxine en réponse à un niveau élevé de GDF-15,
la sérélaxine étant administrée par voie sous-cutanée à un débit de perfusion de 3 µg/kg/jour à 150 µg/kg/jour pour maintenir une concentration sérique de 1 ng/ml à 100 ng/ml chez le patient.

2. Sérélaxine pour une utilisation selon la revendication 1, la charge pulmonaire étant définie comme la pression capillaire pulmonaire (PCP), la pression artérielle pulmonaire (PAP) moyenne ou la résistance vasculaire pulmonaire (RVP).

3. Sérélaxine pour une utilisation selon l'une quelconque des revendications précédentes, la sérélaxine étant administrée pour maintenir une concentration sérique de 10 ng/ml chez le patient.

4. Sérélaxine pour une utilisation selon l'une quelconque des revendications précédentes, la sérélaxine étant administrée par voie sous-cutanée à un débit de perfusion de 30 µg/kg/jour.

5. Sérélaxine pour une utilisation selon l'une quelconque des revendications précédentes, la sérélaxine étant administrée en continu pendant au moins 24 heures.

6. Sérélaxine pour une utilisation dans un procédé de détermination du pronostic de mortalité d'un patient présentant une insuffisance cardiaque aiguë (ICA) comprenant :
l'obtention d'un premier échantillon biologique d'un patient,
la détection du niveau de GDF-15 dans le premier échantillon biologique ;
l'administration d'une quantité thérapeutiquement efficace de sérélaxine, la sérélaxine étant administrée par voie sous-cutanée à un débit de perfusion de 3 µg/kg/jour à 150 µg/kg/jour pour maintenir une concentration sérique de 1 ng/ml à 100 ng/ml chez le patient ;
l'obtention d'un second échantillon biologique du patient après administration de sérélaxine ;
la détection du niveau de GDF-15 dans le second échantillon biologique ;
la comparaison du niveau de GDF-15 dans le premier échantillon biologique et dans le second échantillon biologique ; et
la prédiction de la probabilité de survie, un niveau réduit de GDF-15 dans le second échantillon biologique prédisant une probabilité accrue de survie.

7. Sérélaxine pour une utilisation selon la revendication 6, le second échantillon biologique étant obtenu 2 ou 14 jours après administration de sérélaxine.

8. Sérélaxine pour une utilisation selon l'une quelconque des revendications 6 et 7, la sérélaxine étant administrée pour maintenir une concentration sérique de 10 ng/ml chez le patient.

9. Sérélaxine pour une utilisation selon l'une quelconque des revendications 6 à 8, la sérélaxine étant administrée par voie sous-cutanée à un débit de perfusion de 30 µg/kg/jour.

10. Sérélaxine pour une utilisation selon l'une quelconque des revendications 6 à 9, la sérélaxine étant administrée en continu pendant au moins 24 heures.
